# EUROPEAN PATENT APPLICATION

(11) **EP 4 567 427 A1**
(43) Date of publication of application: **11.06.2025**
(21) Application number: 23858985.7
(22) Date of filing: 24.07.2023
(51) Int. Cl.: G01N 33/68

(54) **BIOMARKER AND RELATED DETECTION KIT FOR ALZHEIMER'S DISEASE**

(30) Priority: 01.09.2022 CN 202211065261
(71) Applicant: Shanghai Raising Pharmaceutical Co., Ltd., Pudong New District Shanghai 201321 (CN)
(72) Inventor: ZHANG, Huan, Shanghai 201321 (CN)
(74) Representative: Michalski Hüttermann & Partner Patentanwälte mbB
(86) International application number: PCT/CN2023/108805
(87) International publication number: WO 2024/045949

(57) **Abstract**

A method for distinguishing or differentially diagnosing Alzheimer's disease from other neurodegenerative diseases, comprising determining the level of TPK1 protein in a sample from a subject, wherein a decrease in the level of TPK1 protein compared to a reference value indicates that the subject has Alzheimer's disease. Methods, compositions, test strips, test cards and/or kits for distinguishing or differentially diagnosing Alzheimer's disease from other neurodegenerative diseases by detecting a biomarker, wherein the methods, compositions, test strips, test cards and/or kits can specifically diagnose Alzheimer's disease.

## Description

### Cross-reference to related application

This application claims priority to the Chinese patent application titled "Biomarkers for Alzheimer's Disease and Related Detection Kits" filed on September 1, 2022, with the application number 202211065261.2, the entire contents of which are incorporated herein by reference.

### Technical Field

The present invention relates to the field of medical diagnostics, specifically to use of thiamine pyrophosphokinase 1 (TPK1) as a biomarker for diagnosing Alzheimer's disease. The invention also relates to methods, compositions, test strips, test cards, and/or kits for detecting and diagnosing Alzheimer's disease using this biomarker.

### Background

Alzheimer's disease is a common chronic, progressive neurodegenerative disorder of the central nervous system, clinically characterized by progressive memory decline, cognitive dysfunction, behavioral abnormalities, and social impairment. The condition typically worsens progressively and is irreversible. With the aging of the population, the incidence of Alzheimer's disease is on the rise. According to statistical data, the prevalence of Alzheimer's disease (AD) in the elderly is third only to cardiovascular diseases and cancer, with nearly 36 million people worldwide suffering from AD.

The pathogenesis of Alzheimer's disease remains unclear. Current theories include molecular genetics and gene theory, inflammation theory, free radical theory, cholinergic theory, metal ion hypothesis, viral theory, oxidative stress theory, and estrogen level reduction theory. Most research is of uncertainty, unable to distinguish which mechanisms are causes and which are effects. Therefore, many research institutes and universities have invested significant resources in studying the pathogenesis of AD, but with limited success.

Due to the complexity of neural cells and limited sampling, screening for age-related genes at the gene expression level has become the best approach for studying the molecular pathogenesis of Alzheimer's disease. Currently, there are few biomarkers for diagnosing AD, and their sensitivity and specificity are not ideal, with only a few being used as auxiliary diagnostic methods in clinical practice. Therefore, finding new biomarkers for the clinical detection and treatment of Alzheimer's disease can help improve diagnostic sensitivity and specificity, as well as enable personalized treatment for Alzheimer's.

### Summary of the Invention

The present invention aims to provide a biomarker for diagnosing Alzheimer's disease, particularly for distinguishing or differentially diagnosing Alzheimer's disease from other neurodegenerative diseases, with the goal of providing an objective diagnostic basis for clinically diagnosing Alzheimer's disease, especially for distinguishing it from other neurodegenerative diseases.

In one aspect, the invention provides a method for distinguishing or differentially diagnosing Alzheimer's disease from other neurodegenerative diseases, comprising determining the level of TPK1 protein in a sample from a subject and comparing it to a reference value, wherein the subject is suspected of having a neurodegenerative disease but it is difficult to determine whether the neurodegenerative disease is Alzheimer's disease, and wherein a decrease in the level of TPK1 protein compared to the reference value suggests that the subject has Alzheimer's disease.

In another aspect, the invention provides an in vitro method for detecting the level of TPK1 protein, comprising: (a) collecting a sample from a subject suspected of having a neurodegenerative disease but difficult to determine whether the neurodegenerative disease is Alzheimer's disease, (b) measuring the level of TPK1 protein in the sample and comparing it to a reference value, wherein a decrease in the level of TPK1 protein compared to the reference value suggests that the subject has Alzheimer's disease.

In yet another aspect, the invention provides a composition for distinguishing or differentially diagnosing Alzheimer's disease from other neurodegenerative diseases, comprising a reagent capable of detecting the level of TPK1 protein in a sample from a subject, wherein the subject is suspected of having a neurodegenerative disease but it is difficult to determine whether the neurodegenerative disease is Alzheimer's disease, and wherein the reagent detects a decrease in the level of TPK1 protein compared to a reference value, suggesting that the subject has Alzheimer's disease.

In a further aspect, the invention provides a kit for distinguishing or differentially diagnosing Alzheimer's disease from other neurodegenerative diseases, comprising a reagent for detecting the level of TPK1 protein in a sample from a subject, wherein the subject is suspected of having a neurodegenerative disease but it is difficult to determine whether the neurodegenerative disease is Alzheimer's disease, and wherein the kit further includes instructions or inserts indicating that a decrease in the level of TPK1 protein compared to a reference value suggests that the subject has Alzheimer's disease.

In yet another aspect, the invention provides a kit for detecting the level of TPK1 protein, comprising a reagent for detecting the level of TPK1 protein in a sample from a subject, wherein the subject is suspected of having a neurodegenerative disease but it is difficult to determine whether the neurodegenerative disease is Alzheimer's disease, and wherein the kit further includes instructions or inserts indicating that a decrease in the level of TPK1 protein compared to a reference value suggests that the subject has Alzheimer's disease.

In another aspect, the invention provides a test strip or test card for distinguishing or differentially diagnosing Alzheimer's disease from other neurodegenerative diseases, wherein the test strip or test card comprises a portion for receiving a sample from a subject and a portion coated with a reagent capable of specifically binding to a biomarker, wherein the subject is suspected of having a neurodegenerative disease but it is difficult to determine whether the neurodegenerative disease is Alzheimer's disease, and wherein the biomarker is TPK1 protein, and a decrease in the level of TPK1 protein compared to a reference value suggests that the subject has Alzheimer's disease.

In yet another aspect, the invention provides the use of a reagent for detecting a biomarker in a sample from a subject in the preparation of a test strip, test card, and/or kit for distinguishing or differentially diagnosing Alzheimer's disease from other neurodegenerative diseases, wherein the subject is suspected of having a neurodegenerative disease but it is difficult to determine whether the neurodegenerative disease is Alzheimer's disease, and wherein the biomarker is TPK1 protein, and a decrease in the level of TPK1 protein compared to a reference value suggests that the subject has Alzheimer's disease.

In another aspect, the invention provides a method for treating a subject with Alzheimer's disease, comprising the steps of: (a) diagnosing a subject suspected of having Alzheimer's disease using the method, composition, test strip, test card, and/or kit of the invention, and (b) administering a therapeutically effective amount of a drug for treating Alzheimer's disease or performing a therapy related to Alzheimer's disease.

In yet another aspect, the invention provides a method for determining the likelihood that a subject has Alzheimer's disease, wherein the subject is suspected of having a neurodegenerative disease but it is difficult to determine whether the neurodegenerative disease is Alzheimer's disease, wherein the method comprises determining the level of TPK1 protein in a sample from the subject and comparing it to a reference value, wherein a decrease in the level of TPK1 protein compared to the reference value indicates that the subject is more likely to have Alzheimer's disease than other neurodegenerative diseases.

In another aspect, the invention provides a method for determining the likelihood that a subject has a neurodegenerative disease other than Alzheimer's disease, wherein the subject is suspected of having a neurodegenerative disease but it is difficult to exclude that the neurodegenerative disease is not Alzheimer's disease, wherein the method comprises determining the level of TPK1 protein in a sample from the subject and comparing it to a reference value, wherein the level of TPK1 protein remaining unchanged or increased compared to the reference value indicates that the subject is less likely to have Alzheimer's disease.

In yet another aspect, the invention provides an immunoassay composition, comprising TPK1 protein from a sample of a subject and an effective amount of a reagent (preferably a binding partner) for detecting the level of TPK1 protein, and/or comprising a complex formed by TPK1 protein from a sample of a subject and an effective amount of a reagent (preferably a binding partner) for detecting the level of TPK1 protein, wherein the subject is suspected of having a neurodegenerative disease but it is difficult to determine whether the neurodegenerative disease is Alzheimer's disease, and wherein a decrease in the level of TPK1 protein in the immunoassay composition compared to a reference value suggests that the subject has Alzheimer's disease.

In another aspect, the invention provides a container comprising an immunoassay composition, wherein the immunoassay composition comprises TPK1 protein from a sample of a subject and an effective amount of a reagent (preferably a binding partner) for detecting the level of TPK1 protein, and/or comprising a complex formed by TPK1 protein from a sample of a subject and an effective amount of a reagent (preferably a binding partner) for detecting the level of TPK1 protein, wherein the subject is suspected of having a neurodegenerative disease but it is difficult to determine whether the neurodegenerative disease is Alzheimer's disease, and wherein a decrease in the level of TPK1 protein in the container compared to a reference value suggests that the subject has Alzheimer's disease.

The methods, compositions, test strips, test cards, and/or kits of the invention can specifically distinguish Alzheimer's disease from other neurodegenerative diseases, providing high specificity for the diagnosis of Alzheimer's disease and offering an objective basis for its diagnosis.

### Description of the Drawings

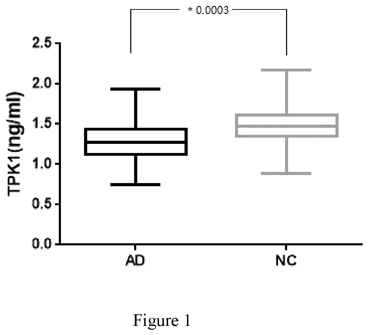

Figure 1: hTPK1 protein detection results.

### Detailed Description

### A. Definitions

Unless otherwise defined, all technical and scientific terms used herein have the same meaning as commonly understood by one of ordinary skill in the art to which this invention belongs. References to techniques used herein are intended to refer to techniques commonly understood in the art, including variations or equivalents of such techniques that are obvious to those skilled in the art. Although the following terms are believed to be well understood by those skilled in the art, the following definitions are provided to better explain the invention.

As used herein, the term "biomarker," also referred to as a "biological marker," refers to a measurable indicator of the biological state of a subject. In this context, the biomarker for distinguishing Alzheimer's disease from other neurodegenerative diseases is a protein from a sample of a subject, rather than a nucleic acid (e.g., mRNA or DNA). In some embodiments, the biomarker is TPK1 protein. In some embodiments, the biomarker is not a nucleic acid encoding TPK1 protein, such as DNA or mRNA encoding TPK1 protein.

As used herein, the names of biomarkers for distinguishing Alzheimer's disease from other neurodegenerative diseases refer to the definitions in the UniProt protein database. In some embodiments, the following proteins refer to proteins from the species Homo sapiens (Human).

The term "TPK1" refers to thiamine pyrophosphokinase 1 (TPK1). The human TPK1 protein is identified in the UniProt protein database with the accession number Q9H3S4, specifically at https://beta.uniprot.org/uniprotkb/Q9H3S4/entry. TPK1, its full name, and its abbreviation are used interchangeably herein.

Clinical diagnosis of AD primarily involves high-resolution imaging of brain tissue using neuroimaging and neuropsychological assessments of patients, with reference to the Diagnostic and Statistical Manual of Mental Disorders-IV (DSM-IV) published by the American Psychiatric Association. Unless otherwise specified, the diagnosis of AD in this document follows the above standard.

Neurodegenerative diseases are characterized by the significant loss of specific neurons (gradual loss of neuronal structure and function), and common neurodegenerative diseases include, for example, Alzheimer's disease (AD), Parkinson's disease (PD), Huntington's disease (HD), Creutzfeldt-Jakob disease, multiple sclerosis, primary lateral sclerosis, spinal muscular atrophy, and many others. AD is one of the neurodegenerative diseases, and its pathological features mainly include plaques and tangles in the brain, where the plaques are primarily composed of amyloid-beta protein, and the tangles are mainly composed of hyperphosphorylated tau protein. AD and many other neurodegenerative diseases share overlapping symptoms, making them difficult to distinguish, and their etiologies are complex, with current clinical diagnostic methods often unable to accurately differentiate them. Additionally, other diseases such as cerebral infarction and senile dementia can also cause symptoms similar to AD, making clinical differentiation challenging. The present invention demonstrates that TPK1 protein levels are reduced in AD patients, and more importantly, TPK1 protein levels are not reduced in other neurodegenerative diseases besides AD, making it useful for the differential diagnosis of AD from other neurodegenerative diseases.

As used herein, the terms "diagnose," "diagnosed," or "diagnosing" refer to methods for detecting and/or identifying the disease state of a subject, distinguishing and/or determining whether a subject has a specific disease.

The term "distinguishing or differentially diagnosing Alzheimer's disease from other neurodegenerative diseases" refers to diagnosing Alzheimer's disease by detecting the level of TPK1 protein discovered in the present invention, particularly for diagnosing subjects suspected of having Alzheimer's disease based on clinical manifestations related to neurodegenerative diseases.

In this context, the differential diagnosis of Alzheimer's disease from other neurodegenerative diseases is performed by comparing the level of TPK1 protein in the invention to a reference value, wherein a decrease in the level of TPK1 protein suggests that the subject has Alzheimer's disease. In some embodiments, the reference value is the level of TPK1 protein in a sample from a healthy subject (or a non-AD subject, or a non-AD elderly subject) or from a standard sample. When the level of TPK1 protein in the sample is lower than this reference value, it suggests that the subject has Alzheimer's disease. In one embodiment, the reference value is 2.5 ng/mL-1.0 ng/mL, 2.5 ng/mL-1.0 ng/mL, 2.2 ng/mL-1.0 ng/mL, 2.0 ng/mL-1.0 ng/mL, 1.5 ng/mL-1.0 ng/mL, or 1.5 ng/mL-1.2 ng/mL. In one embodiment, the reference value is 2.2 ng/mL, 2.1 ng/mL, 2.0 ng/mL, 1.9 ng/mL, 1.8 ng/mL, 1.7 ng/mL, 1.6 ng/mL, 1.5 ng/mL, 1.4 ng/mL, 1.3 ng/mL, 1.2 ng/mL, 1.1 ng/mL, 1.0 ng/mL, 0.9 ng/mL, 0.8 ng/mL, 0.7 ng/mL, 0.6 ng/mL, or 0.5 ng/mL.

As used herein, the term "sample" refers to a blood sample isolated from a subject, such as whole blood or blood cells. Specifically, the sample may be a blood sample obtained from a subject suspected of having or having Alzheimer's disease and other neurodegenerative diseases. In some embodiments, the sample is a human whole blood sample. In some embodiments, the sample is a human whole blood cell sample. The sample of the invention does not include blood fractions, such as plasma and serum. The sample of the invention also does not include brain tissue samples from the subject, such as cortical tissue samples from the subject.

As used herein, the term "subject" refers to a mammal, preferably a human. In some embodiments, the subject is one for whom it is difficult to distinguish Alzheimer's disease from other neurodegenerative diseases using current diagnostic standards. In some embodiments, the subject is an elderly subject, optionally an elderly subject aged over 50, such as 55, 56, 57, 58, 59, 60, 61, 62, 63, 64, 65, 66, 67, 68, 69, or 70 years old, and the elderly subject is one for whom it is difficult to distinguish Alzheimer's disease from other neurodegenerative diseases using current diagnostic standards. In some embodiments, the subject is an elderly subject aged 55-90, 55-80, 60-90, 60-80, 55-75, 55-70, 60-70, or 55-60 years old, and the elderly subject is one for whom it is difficult to distinguish Alzheimer's disease from other neurodegenerative diseases using current diagnostic standards.

As used herein, the term "determining the level of TPK1 protein in a sample from a subject" refers to measuring the content of TPK1 protein in a sample from a subject. Methods for measuring protein levels are known in the art and include, but are not limited to, immunoassays, including competitive immunoassays, non-competitive immunoassays, enzyme-linked immunosorbent assays (ELISA), immunohistochemical assays, chemiluminescent assays (preferably magnetic particle chemiluminescence), Western blot assays, and Dot blot assays; and mass spectrometry assays.

Accordingly, as used herein, the term "reagent capable of detecting the level of TPK1 protein" refers to a substance or means/method that can be used to determine whether the level of a biomarker in a sample obtained from a subject is increased, unchanged, or decreased. In this context, the reagent capable of detecting the level of TPK1 protein may be a binding partner that can target the biomarker and bind to it. For example, the binding partner may be an antibody or antigen-binding fragment that can target the biomarker and bind to it. The level of TPK1 protein is detected by a binding partner targeting the biomarker. For example, the level of TPK1 protein is detected by an antibody that specifically binds to this biomarker. Alternatively, the level of TPK1 protein is measured by mass spectrometry or antibody-based immunoassay methods, such as competitive immunoassays, non-competitive immunoassays, enzyme-linked immunosorbent assays (ELISA), immunohistochemical assays, chemiluminescent assays (preferably magnetic particle chemiluminescence), Western blot assays, and Dot blot assays.

The term "antibody" refers to an immunoglobulin molecule containing at least one antigen-binding site and capable of specifically binding to an antigen. For example, the antibody may be a monoclonal antibody, polyclonal antibody, antibody fragment, humanized antibody, camelid antibody, chimeric antibody, etc. The antibody may be further modified to carry a detectable label, such as a label detectable by chemiluminescence.

As used herein, unless the context clearly indicates otherwise, the singular forms "a," "an," and "the" include plural references.

As used herein, the terms "optionally," "optional," or "optionally" mean that the subsequently described event or circumstance may or may not occur. For example, "optionally, the test strip or test card further comprises a detection portion for detecting whether the biomarker binds to the reagent, and/or a portion for quality control" means that the test strip or test card may further comprise a detection portion for detecting whether the biomarker binds to the reagent, and/or a portion for quality control; or this situation may not exist.

As used herein, the terms "comprising," "including," "having," or "containing" and their variants in this context are inclusive or open-ended and do not exclude other unlisted elements or method steps.

### B. Methods for Distinguishing or Differentially Diagnosing Alzheimer's Disease and Other Neurodegenerative Diseases

The level of TPK1 protein in a blood sample from a subject in the present invention can be used to specifically distinguish Alzheimer's disease from other neurodegenerative diseases, providing high specificity for the diagnosis of Alzheimer's disease.

Without being bound by any theory, thiamine pyrophosphokinase 1 (TPK1) catalyzes the phosphorylation of thiamine to thiamine diphosphate (TDP). TDP is a coenzyme for key enzymes (pyruvate dehydrogenase, α-ketoglutarate dehydrogenase, and transketolase) in the glucose metabolism process in human cells, and a decrease in TDP levels will lead to impaired glucose metabolism in cells. Impaired brain glucose metabolism is a constant and major pathophysiological feature of AD, closely associated with the degree of cognitive impairment and disease progression. The applicant has found that in AD patients, the inhibition of TPK1 expression leads to a decrease in TDP levels, and the measurement of TPK1 protein levels can be used for the clinical diagnosis of Alzheimer's disease.

In one aspect, the invention provides a method for distinguishing or differentially diagnosing Alzheimer's disease from other neurodegenerative diseases, comprising determining the level of TPK1 protein in a sample from a subject and comparing it to a reference value, wherein the subject is suspected of having a neurodegenerative disease but it is difficult to determine whether the neurodegenerative disease is Alzheimer's disease, and wherein a decrease in the level of TPK1 protein compared to the reference value suggests that the subject has Alzheimer's disease.

In another aspect, the invention also provides a method for determining the likelihood that a subject has Alzheimer's disease, wherein the subject is suspected of having a neurodegenerative disease but it is difficult to determine whether the neurodegenerative disease is Alzheimer's disease, wherein the method comprises determining the level of TPK1 protein in a sample from the subject and comparing it to a reference value, wherein a decrease or equivalent level of TPK1 protein compared to the reference value indicates that the subject is more likely to have a depressive disorder than Alzheimer's disease.

In another aspect, the invention also provides an in vitro method for detecting the level of TPK1 protein, comprising: (a) collecting a sample from a subject suspected of having a neurodegenerative disease but difficult to determine whether the neurodegenerative disease is Alzheimer's disease, (b) measuring the level of TPK1 protein in the sample and comparing it to a reference value, wherein a decrease in the level of TPK1 protein compared to the reference value suggests that the subject has Alzheimer's disease.

In some embodiments, the reference value is the level of TPK1 protein in a sample from a healthy subject (or a non-AD subject, or a non-AD elderly subject) or from a standard sample.

In some embodiments, the sample is a blood sample. In some embodiments, the sample is a human whole blood sample. In some embodiments, the sample is a human whole blood cell sample. In some embodiments, the sample is a blood sample obtained from a subject suspected of having or having Alzheimer's disease and other neurodegenerative diseases, preferably a human whole blood sample, more preferably a human whole blood cell sample.

In some embodiments, the level of TPK1 protein is determined by a method selected from: immunoassays, including competitive immunoassays, non-competitive immunoassays, enzyme-linked immunosorbent assays (ELISA), immunohistochemical assays, chemiluminescent assays (preferably magnetic particle chemiluminescence), Western blot assays, and Dot blot assays; mass spectrometry assays.

The methods for immunoassays are known in the art and can be found, for example, in (Tijssen, Practice and Theory of Enzyme Immunoassays (Elsevier, Amsterdam, 1985); Campbell, Monoclonal Antibody Technology (Elsevier, Amsterdam, 1984); Hurrell, Monoclonal Hybridoma Antibodies: Techniques and Applications (CRC Press, Boca Raton, FL, 1982); and Zola, Monoclonal Antibodies: A Manual of Techniques, pp. 147-158 (CRC Press, Inc., 1987)).

The methods for mass spectrometry assays are also known in the art and can be found, for example, in A review on mass spectrometry-based quantitative proteomics: Targeted and data independent acquisition, Anal Chim Acta. 2017 Apr 29; 964:7-23. doi: 10.1016/j.aca.2017.01.059.

In some embodiments, the level of TPK1 protein is detected by a reagent capable of specifically binding to the biomarker; optionally, the level of TPK1 protein is detected by a binding partner targeting the biomarker; optionally, the level of TPK1 protein is detected by an antibody targeting the biomarker; optionally, the antibody is selected from monoclonal antibodies, polyclonal antibodies, antibody fragments, humanized antibodies, camelid antibodies, and chimeric antibodies.

Methods for preparing antibodies, such as monoclonal antibodies, are known to those skilled in the art and can be found in Clackson et al., Nature 352, 624-628

(1991) and Marks et al., J. Mol. Biol. 222, 581-597 (1991). Antibodies targeting TPK1 protein in the present invention are also commercially available, for example, antibodies against TPK1 protein can be purchased from Abcam (recombinant anti-TPK1 antibody, ab170863) or Sino Biological (TPK1 polyclonal antibody, 15390-T16).

In some embodiments, the reagent may be further modified to carry a detectable label. In some embodiments, the detectable label may include, for example, horseradish peroxidase (HRP), alkaline phosphatase (AP), glucose oxidase, radioactive isotopes, fluorescent reporters, etc. Optionally, the antibody may be further modified to carry a detectable label, such as a label detectable by chemiluminescence. In some embodiments, the antibody is a magnetic particle-labeled antibody targeting TPK1 protein. In some embodiments, the antibody is a biotin-labeled antibody targeting TPK1 protein. In some embodiments, the antibody comprises a combination of a magnetic particle-labeled antibody targeting TPK1 protein and a biotin-labeled antibody targeting TPK1 protein.

In some embodiments, the subject is one for whom it is difficult to distinguish Alzheimer's disease from other neurodegenerative diseases using current diagnostic standards. In some embodiments, the subject is an elderly subject, optionally an elderly subject aged over 50, such as 55, 56, 57, 58, 59, 60, 61, 62, 63, 64, 65, 66, 67, 68, 69, or 70 years old, and the elderly subject is one for whom it is difficult to distinguish Alzheimer's disease from other neurodegenerative diseases using current diagnostic standards. In some embodiments, the subject is an elderly subject aged 55-90, 55-80, 60-90, 60-80, 55-75, 55-70, 60-70, or 55-60 years old, and the elderly subject is one for whom it is difficult to distinguish Alzheimer's disease from other neurodegenerative diseases using current diagnostic standards.

### C. Compositions, Test Strips, Test Cards, and/or Kits for Distinguishing or Differentially Diagnosing Alzheimer's Disease and Other Neurodegenerative Diseases

In one aspect, the invention also relates to a composition for detecting the level of TPK1 protein, wherein the composition includes a reagent capable of detecting the level of TPK1 protein in a sample from a subject.

In another aspect, the invention also relates to a test strip or test card for distinguishing or differentially diagnosing Alzheimer's disease from other neurodegenerative diseases, wherein the test strip or test card comprises a portion for receiving a sample from a subject and a portion coated with a reagent capable of specifically binding to a biomarker.

In yet another aspect, the invention also relates to a kit for detecting the level of TPK1 protein, or a kit for distinguishing or differentially diagnosing Alzheimer's disease from other neurodegenerative diseases, wherein the kit comprises the reagent of the invention for detecting the level of TPK1 protein in a sample from a subject, or the kit comprises the test strip or test card of the invention.

In another aspect, the invention also relates to an immunoassay composition, wherein the composition comprises a biomarker from a sample of a subject and an effective amount of a reagent (preferably a binding partner) for detecting the level of TPK1 protein, and/or comprises a complex formed by a biomarker from a sample of a subject and an effective amount of a reagent (preferably a binding partner) for detecting the level of TPK1 protein, wherein the subject is suspected of having a neurodegenerative disease but it is difficult to determine whether the neurodegenerative disease is Alzheimer's disease.

In yet another aspect, the invention also relates to a container comprising an immunoassay composition, wherein the immunoassay composition comprises a biomarker from a sample of a subject and an effective amount of a reagent (preferably a binding partner) for detecting the level of TPK1 protein, and/or comprises a complex formed by a biomarker from a sample of a subject and an effective amount of a reagent (preferably a binding partner) for detecting the level of TPK1 protein, wherein the subject is suspected of having a neurodegenerative disease but it is difficult to determine whether the neurodegenerative disease is Alzheimer's disease.

In some embodiments, the subject is suspected of having a neurodegenerative disease but difficult to determine whether the neurodegenerative disease is Alzheimer's disease. In some embodiments, the subject is an elderly subject, optionally an elderly subject aged over 50, such as 55, 56, 57, 58, 59, 60, 61, 62, 63, 64, 65, 66, 67, 68, 69, or 70 years old, and the elderly subject is one for whom it is difficult to distinguish Alzheimer's disease from other neurodegenerative diseases using current diagnostic standards. In some embodiments, the subject is an elderly subject aged 55-90, 55-80, 60-90, 60-80, 55-75, 55-70, 60-70, or 55-60 years old, and the elderly subject is one for whom it is difficult to distinguish Alzheimer's disease from other neurodegenerative diseases using current diagnostic standards.

In some embodiments, a decrease in the level of TPK1 protein compared to a reference value suggests that the subject has Alzheimer's disease.

In some embodiments, the reference value is the level of TPK1 protein in a sample from a healthy subject (or a non-AD subject, or a non-AD elderly subject) or from a standard sample.

In some embodiments, the sample is a blood sample. In some embodiments, the sample is a human whole blood sample. In some embodiments, the sample is a human whole blood cell sample. In some embodiments, the sample is a blood sample obtained from a subject suspected of having or having Alzheimer's disease and other neurodegenerative diseases, preferably a human whole blood sample, more preferably a human whole blood cell sample.

When the biomarker to be detected is known, methods for preparing antibodies against this biomarker and further preparing test strips and test cards for detecting this biomarker based on the antibody are known to those skilled in the art.

In some embodiments, detection reagents/detection reagent compositions, test strips, test cards, and/or kits can be prepared for the following detections, including but not limited to immunoassays, including competitive immunoassays, non-competitive immunoassays, enzyme-linked immunosorbent assays (ELISA), immunohistochemical assays, chemiluminescent assays (preferably magnetic particle chemiluminescence), Western blot assays, and Dot blot assays; mass spectrometry assays.

In some embodiments, the detection reagents/detection reagent compositions, test strips, test cards, and/or kits can be used for chemiluminescence detection; optionally, the detection reagents/detection reagent compositions, test strips, test cards, and/or kits are compatible with fully automated chemiluminescence detection instruments or automated/digital detection platforms.

The level of TPK1 protein can be detected by a binding partner targeting the biomarker. The reagent for detecting the level of TPK1 protein may be an antibody or a derivative thereof. In some embodiments, the level of TPK1 protein can be detected by a binding partner targeting the biomarker; optionally, the level of TPK1 protein is detected by an antibody targeting the biomarker; optionally, the antibody is selected from monoclonal antibodies, polyclonal antibodies, antibody fragments, humanized antibodies, camelid antibodies, and chimeric antibodies. In some embodiments, the antibody may be further modified to carry a detectable label, such as a label detectable by chemiluminescence.

In some embodiments, the detectable label may include, for example, horseradish peroxidase (HRP), alkaline phosphatase (AP), glucose oxidase, radioactive isotopes, fluorescent reporters, etc. Optionally, the antibody may be further modified to carry a detectable label, such as a label detectable by chemiluminescence. In some embodiments, the antibody is a magnetic particle-labeled antibody targeting TPK1 protein. In some embodiments, the antibody is a biotin-labeled antibody targeting TPK1 protein. In some embodiments, the antibody comprises a combination of a magnetic particle-labeled antibody targeting TPK1 protein and a biotin-labeled antibody targeting TPK1 protein.

In some embodiments, the detection reagents/detection reagent compositions, test strips, test cards, and/or kits comprise the following components: magnetic particle-labeled antibody targeting TPK1 protein, biotin-labeled antibody targeting TPK1 protein, streptavidin-labeled alkaline phosphatase complex, alkaline phosphatase substrate, buffer, diluent, standard, calibrator, and control.

In some embodiments, in the test strip/test card or a kit comprising the test strip/test card, the magnetic particle-labeled antibody targeting TPK1 protein is a magnetic particle-labeled monoclonal antibody targeting TPK1 protein, with a concentration of 80 µL/strip, wherein the antibody concentration is 0.5 mg/mL. In some embodiments, for each single-use test strip/test card, 80 µL/strip of magnetic particle-labeled monoclonal antibody targeting TPK1 protein is added, with an antibody concentration of 0.5 mg/mL. In some embodiments, in the test strip/test card or a kit comprising the test strip/test card, the biotin-labeled antibody targeting TPK1 protein is a biotin-labeled monoclonal antibody targeting TPK1 protein, with a concentration of 80 µL/strip. In some embodiments, in the test strip/test card or a kit comprising the test strip/test card, the streptavidin-labeled alkaline phosphatase complex has a concentration of 80 µL/strip. In some embodiments, in the test strip/test card or a kit comprising the test strip/test card, the alkaline phosphatase substrate has a concentration of 200 µL/strip. In some embodiments, in the test strip/test card or a kit comprising the test strip/test card, the buffer is 0.01M PBS buffer containing 0.08% Tween 20 and 0.05% proclin 300, 2 mL/strip. In some embodiments, in the test strip/test card or a kit comprising the test strip/test card, the diluent is 0.01M PBS buffer containing 0.08% Tween 20 and 0.05% proclin 300. In some embodiments, in the test strip/test card or a kit comprising the test strip/test card, the calibrator is recombinant TPK1 protein, with a concentration of 5 ng/mL. In some embodiments, in the test strip/test card or a kit comprising the test strip/test card, the standard is recombinant TPK1 protein, with a concentration of 0.02-40 ng/mL in 6 gradient dilutions, i.e., 40 ng/mL, 20 ng/mL, 4 ng/mL, 2 ng/mL, 0.8 ng/mL, and 0.2 ng/mL. In some embodiments, in the test strip/test card or a kit comprising the test strip/test card, the control is recombinant TPK1 protein, and the standard is recombinant TPK1 protein. The standard is used to establish the main curve of the test strip/test card/kit and to correct the working curve, with gradients set at 40 ng/mL, 20 ng/mL, 4 ng/mL, 2 ng/mL, 0.8 ng/mL, and 0.2 ng/mL. The control can be used as a reference for the reliability of the experimental results, and its measured value should be within the target range of the control, with the specific concentration adjusted according to the antibody prepared in different batches.

### Examples

The following examples further illustrate the invention by way of example. It should be understood that the invention is not limited to any of the examples described below.

### Example 1 - Preparation of a Thiamine Pyrophosphokinase 1 (TPK1) Magnetic Particle Chemiluminescence Assay Kit [0080] 1. Main components are shown in Table 1:

**Table 1**

| Composition | Main components and content |
|---|---|
| 1. Reagent | Biotin-labeled antibody: 80 µL/strip, antibody concentration 0.5 mg/mL |
| | Magnetic bead-labeled antibody: 80 µL/strip, antibody concentration 0.5 mg/mL |
| | Streptavidin-labeled alkaline phosphatase complex: 80 µL/strip |
| | Alkaline phosphatase substrate: 200 µL/strip |
| | 0.01M PBS buffer containing 0.08% Tween 20 and 0.05% proclin 300, 2 mL/strip |
| 2. Calibrator | Calibrator S1 recombinant TPK1 protein: 5 ng/mL |
| | Standard S2 recombinant TPK1 protein: 0.5 ng/mL |
| | Diluent is 0.01M PBS buffer containing 0.08% Tween 20 and 0.05% proclin 300. |
| 3. Control | Control C1: recombinant TPK1 protein; |
| | Control C2: recombinant TPK1 protein; |
| | Diluent is 0.01M PBS buffer containing 0.08% Tween 20 and 0.05% proclin 300. |

### 2. Instruments

RSClia Fully Automated Chemiluminescence Analyzer (Shanghai Rixin Medical Technology Co., Ltd.)
POClia8 Fully Automated Chemiluminescence Analyzer (Taizhou Zecheng Biotechnology Co., Ltd.)

### 3. Storage Conditions

2-8°C, protected from light, sealed, and not frozen.

### 4. Detection Steps

The kit in this example is used for testing human whole blood samples (collected in K2-EDTA or lithium heparin tubes), and the specific operating steps are as follows.

### 4.1 Pre-detection Preparation

(1) Before detection, take out the kit and let it equilibrate at room temperature, and preheat the instrument for at least 30 minutes.
(2) Add the whole blood sample to the sample diluent at a ratio of 1:40 (e.g., 10 µL of sample to 400 µL of sample diluent), vortex mix thoroughly for more than 30 seconds, and set aside.
(3) Main curve input: If there is no reagent batch curve available in the instrument, scan the main curve card, read the standard curve data, and the instrument will automatically fit the standard curve.

4.2 Calibration: Adjust each calibration point on the pre-defined main curve to a new, instrument-optimized measurement level by measuring the high and low calibrators in the kit, i.e., the working curve.

### 4.3 Quality Control

(1) Use the high and low controls in the kit, and perform control testing at least once every 24 hours. Control testing must also be performed after each reagent kit replacement or calibration.
(2) Each laboratory can set appropriate control ranges and control cycles according to their own conditions. The measured values of the controls must fall within the specified control range.
(3) If the control results are within the target range, the results are valid; otherwise, appropriate corrective measures must be taken.

### 4.4 Reagent and Sample Loading

(1) Before loading the reagents, visually inspect the reagent solutions to ensure they are clear, free of foreign matter, precipitates, or flocculent substances. Also, check that the liquid is at the bottom of the tube; if not, gently shake it to the bottom.
(2) Follow the system operation instructions, scan the reagent barcode, and complete the loading of the reagent strips.
(3) Open the instrument front cover and insert the required reagent strips into the reagent slots.
(4) Mix the calibrators, controls, or diluted samples thoroughly, then transfer them to the corresponding sample wells of the reagent strips using a pipette or quantitative pipette.
(5) After adding the samples, close the instrument front cover and prepare for testing.

4.5 Testing Procedure: The detection reaction process and related parameters for different products have been pre-defined in the instrument operation software. After setting the parameters according to the instrument operation instructions, click the start button, and the instrument will automatically begin testing.

4.6 Result Output: The analyzer automatically calculates the TPK1 concentration for each sample based on the working curve obtained from the standard curve after two-point calibration. The concentration is expressed in ng/mL.

### 5. Performance Evaluation

(1) Physical Performance: The reagent solutions should be clear, free of foreign matter, precipitates, or flocculent substances; the kit packaging labels should be clear, not worn, and easy to identify.
(2) Limit of Detection: Not higher than 0.01 ng/mL.
(3) Linear Range: 0.01 ng/mL to 10 ng/mL, with a linear correlation coefficient r ≥ 0.990.
(4) Precision: Intra-assay precision ≤ 10%, inter-assay precision ≤ 15%.
(5) Accuracy: The recovery rate of the accuracy reference material should be within the range of 85%-115%.

### Example 2 - Detection of Different Populations Using the TPK1 Magnetic Particle Chemiluminescence Assay Kit

In the following experiments, the TPK1 magnetic particle chemiluminescence assay kit described above was used to detect whole blood samples from healthy individuals (NC) and Alzheimer's disease patients (AD).

**Table 2**

| Population | Number and Average Age | Sample Type |
|---|---|---|
| Healthy (NC) | 101 cases; age range 20-81 | Whole blood |
| | Average age 47.5 years | |
| AD Patients (AD) | 31 cases; age range 57-89 | Whole blood |
| | Average age 69.5 years | |

### Procedure:

(1) Calibrator calibration
(2) Sample dilution and testing
(3) Result analysis

hTPK1 Protein Detection Results are shown in the below Figure 1 and Table 3.

**Table 3**

| Group | | case number | range | minimum value | maximum value | mean | standard deviation |
|---|---|---|---|---|---|---|---|
| NC | Vp | 101 | 1.294 | 0.836 | 2.13 | 1.44550 | 0.217201 |
| | Number of valid cases | 101 | | | | | |
| AD | Vp | 31 | 1.185 | 0.751 | 1.936 | 1.27674 | 0.243892 |
| | Number of valid cases | 31 | | | | | |

As shown in Figure 1, the mean hTPK1 protein expression in the healthy group (NC) was higher than that in the AD group. Analysis using GraphPad Prism 6.0 showed a significant difference between the healthy group (NC) and the AD group (P-value = 0.0003).

Using the ROC curve method for cutoff value analysis, the maximum AUC cutoff point specificity and sensitivity for the AD group and the control NC group were 62.38% and 61.29%, respectively.

Conclusion: In terms of TPK1 protein levels, the healthy NC group (1.445, min 0.836 - max 2.13) > the elderly AD group (1.277, min 0.751 - max 1.936). There was a significant difference between the healthy NC group and the elderly AD group, suggesting that TPK1 protein can serve as a diagnostic marker for AD, especially for elderly subjects.

The invention further includes the following clause sets:
Clause set A:
   A1. A method for distinguishing or differentially diagnosing Alzheimer's disease from other neurodegenerative diseases, comprising determining the level of TPK1 protein in a sample from a subject and comparing it to a reference value, wherein the subject is suspected of having a neurodegenerative disease but it is difficult to determine whether the neurodegenerative disease is Alzheimer's disease, and wherein a decrease in the level of TPK1 protein compared to the reference value suggests that the subject has Alzheimer's disease.
   A2. The method according to clause A1, wherein the sample is a blood sample, preferably a human whole blood sample, more preferably a human whole blood cell sample.
   A3. The method according to any one of clauses A1-A2, wherein the reference value is the level of TPK1 protein in a sample from a healthy subject (or a non-AD subject, or a non-AD elderly subject) or from a standard sample.
   A4. The method according to any one of clauses A1-A3, wherein the level of TPK1 protein is determined by a method selected from: immunoassays, including competitive immunoassays, non-competitive immunoassays, enzyme-linked immunosorbent assays (ELISA), immunohistochemical assays, chemiluminescent assays (preferably magnetic particle chemiluminescence), Western blot assays, and Dot blot assays; mass spectrometry assays; optionally, the level of TPK1 protein is determined by chemiluminescent assays (preferably magnetic particle chemiluminescence).
   A5. The method according to any one of clauses A1-A4, wherein the level of TPK1 protein is detected by a reagent capable of specifically binding to TPK1 protein; optionally, the level of TPK1 protein is detected by a binding partner that targets TPK1 protein; optionally, the level of TPK1 protein is detected by an antibody that targets TPK1 protein; optionally, the antibody is selected from monoclonal antibodies, polyclonal antibodies, antibody fragments, humanized antibodies, camelid antibodies, and chimeric antibodies.
   A6. The method according to clause A5, wherein the reagent may be further modified to carry a detectable label; optionally, the antibody may be further modified to carry a detectable label, such as a label detectable by chemiluminescence (e.g., a magnetic particle-labeled antibody targeting TPK1 protein, a biotin-labeled antibody targeting TPK1 protein, etc.).
Clause set B:
   B1. An in vitro method for detecting the level of TPK1 protein, comprising: (a) collecting a sample from a subject suspected of having a neurodegenerative disease but difficult to determine whether the neurodegenerative disease is Alzheimer's disease, (b) measuring the level of TPK1 protein in the sample and comparing it to a reference value, wherein a decrease in the level of TPK1 protein compared to the reference value suggests that the subject has Alzheimer's disease.
   B2. The method according to clause B1, wherein the sample is a blood sample, preferably a human whole blood sample, more preferably a human whole blood cell sample.
   B3. The method according to any one of clauses B1-B2, wherein the reference value is the level of TPK1 protein in a sample from a healthy subject (or a non-AD subject, or a non-AD elderly subject) or from a standard sample.
   B4. The method according to any one of clauses B1-B3, wherein the level of TPK1 protein is determined by a method selected from: immunoassays, including competitive immunoassays, non-competitive immunoassays, enzyme-linked immunosorbent assays (ELISA), immunohistochemical assays, chemiluminescent assays (preferably magnetic particle chemiluminescence), Western blot assays, and Dot blot assays; mass spectrometry assays; optionally, the level of TPK1 protein is determined by chemiluminescent assays (preferably magnetic particle chemiluminescence).
   B5. The method according to any one of clauses B1-B4, wherein the level of TPK1 protein is detected by a reagent capable of specifically binding to TPK1 protein; optionally, the level of TPK1 protein is detected by a binding partner that targets TPK1 protein; optionally, the level of TPK1 protein is detected by an antibody that targets TPK1 protein; optionally, the antibody is selected from monoclonal antibodies, polyclonal antibodies, antibody fragments, humanized antibodies, camelid antibodies, and chimeric antibodies.
   B6. The method according to clause B5, wherein the reagent may be further modified to carry a detectable label; preferably, the antibody may be further modified to carry a detectable label, such as a label detectable by chemiluminescence (e.g., a magnetic particle-labeled antibody targeting TPK1 protein, a biotin-labeled antibody targeting TPK1 protein, etc.).
Clause set C:
   C1. A composition for distinguishing or differentially diagnosing Alzheimer's disease from other neurodegenerative diseases, comprising a reagent capable of detecting the level of TPK1 protein in a sample from a subject, wherein the subject is suspected of having a neurodegenerative disease but it is difficult to determine whether the neurodegenerative disease is Alzheimer's disease, and wherein the reagent detects a decrease in the level of TPK1 protein compared to a reference value, suggesting that the subject has Alzheimer's disease.
   C2. The composition according to clause C1, wherein the sample is a blood sample, preferably a human whole blood sample, more preferably a human whole blood cell sample.
   C3. The composition according to any one of clauses C1-C2, wherein the reference value is the level of TPK1 protein in a sample from a healthy subject (or a non-AD subject, or a non-AD elderly subject) or from a standard sample.
   C4. The composition according to any one of clauses C1-C3, wherein the level of TPK1 protein is determined by a method selected from: immunoassays, including competitive immunoassays, non-competitive immunoassays, enzyme-linked immunosorbent assays (ELISA), immunohistochemical assays, chemiluminescent assays (preferably magnetic particle chemiluminescence), Western blot assays, and Dot blot assays; mass spectrometry assays; optionally, the level of TPK1 protein is determined by chemiluminescent assays (preferably magnetic particle chemiluminescence).
   C5. The composition according to any one of clauses C1-C4, wherein the level of TPK1 protein is detected by a reagent capable of specifically binding to TPK1 protein; optionally, the level of TPK1 protein is detected by a binding partner that targets TPK1 protein; optionally, the level of TPK1 protein is detected by an antibody that targets TPK1 protein; optionally, the antibody is selected from monoclonal antibodies, polyclonal antibodies, antibody fragments, humanized antibodies, camelid antibodies, and chimeric antibodies.
   C6. The composition according to clause C5, wherein the reagent may be further modified to carry a detectable label; preferably, the antibody may be further modified to carry a detectable label, such as a label detectable by chemiluminescence (e.g., a magnetic particle-labeled antibody targeting TPK1 protein, a biotin-labeled antibody targeting TPK1 protein, etc.).
Clause set D:
   D1. A kit for distinguishing or differentially diagnosing Alzheimer's disease from other neurodegenerative diseases, comprising a reagent for detecting the level of TPK1 protein in a sample from a subject, wherein the subject is suspected of having a neurodegenerative disease but it is difficult to determine whether the neurodegenerative disease is Alzheimer's disease, and wherein the kit further includes instructions or inserts indicating that a decrease in the level of TPK1 protein compared to a reference value suggests that the subject has Alzheimer's disease.
   D2. The kit according to clause D1, wherein the sample is a blood sample, preferably a human whole blood sample, more preferably a human whole blood cell sample.
   D3. The kit according to any one of clauses D1-D2, wherein the reference value is the level of TPK1 protein in a sample from a healthy subject (or a non-AD subject, or a non-AD elderly subject) or from a standard sample.
   D4. The kit according to any one of clauses D1-D3, wherein the level of TPK1 protein is determined by a method selected from: immunoassays, including competitive immunoassays, non-competitive immunoassays, enzyme-linked immunosorbent assays (ELISA), immunohistochemical assays, chemiluminescent assays (preferably magnetic particle chemiluminescence), Western blot assays, and Dot blot assays; mass spectrometry assays; optionally, the level of TPK1 protein is determined by chemiluminescent assays (preferably magnetic particle chemiluminescence).
   D5. The kit according to any one of clauses D1-D4, wherein the level of TPK1 protein is detected by a reagent capable of specifically binding to TPK1 protein; optionally, the level of TPK1 protein is detected by a binding partner that targets TPK1 protein; optionally, the level of TPK1 protein is detected by an antibody that targets TPK1 protein; optionally, the antibody is selected from monoclonal antibodies, polyclonal antibodies, antibody fragments, humanized antibodies, camelid antibodies, and chimeric antibodies.
   D6. The kit according to clause D5, wherein the reagent may be further modified to carry a detectable label; preferably, the antibody may be further modified to carry a detectable label, such as a label detectable by chemiluminescence (e.g., a magnetic particle-labeled antibody targeting TPK1 protein, a biotin-labeled antibody targeting TPK1 protein, etc.).
   D7. The kit according to any one of clauses D1-D6, wherein the kit can be used for chemiluminescence detection.
   D8. The kit according to any one of clauses D1-D7, wherein the kit are compatible with fully automated chemiluminescence detection instruments or automated/digital detection platforms.
Clause set E:
   E1. A kit for detecting the level of TPK1 protein, comprising a reagent for detecting the level of TPK1 protein in a sample from a subject, wherein the subject is suspected of having a neurodegenerative disease but it is difficult to determine whether the neurodegenerative disease is Alzheimer's disease, and wherein the kit further includes instructions or inserts indicating that a decrease in the level of TPK1 protein compared to a reference value suggests that the subject has Alzheimer's disease.
   E2. The kit according to clause E1, wherein the sample is a blood sample, preferably a human whole blood sample, more preferably a human whole blood cell sample.
   E3. The kit according to any one of clauses E1-E2, wherein the reference value is the level of TPK1 protein in a sample from a healthy subject (or a non-AD subject, or a non-AD elderly subject) or from a standard sample.
   E4. The kit according to any one of clauses E1-E3, wherein the level of TPK1 protein is determined by a method selected from: immunoassays, including competitive immunoassays, non-competitive immunoassays, enzyme-linked immunosorbent assays (ELISA), immunohistochemical assays, chemiluminescent assays (preferably magnetic particle chemiluminescence), Western blot assays, and Dot blot assays; mass spectrometry assays; optionally, the level of TPK1 protein is determined by chemiluminescent assays (preferably magnetic particle chemiluminescence).
   E5. The kit according to any one of clauses E1-E4, wherein the level of TPK1 protein is detected by a reagent capable of specifically binding to TPK1 protein; optionally, the level of TPK1 protein is detected by a binding partner that targets TPK1 protein; optionally, the level of TPK1 protein is detected by an antibody that targets TPK1 protein; optionally, the antibody is selected from monoclonal antibodies, polyclonal antibodies, antibody fragments, humanized antibodies, camelid antibodies, and chimeric antibodies.
   E6. The kit according to clause E5, wherein the reagent may be further modified to carry a detectable label; preferably, the antibody may be further modified to carry a detectable label, such as a label detectable by chemiluminescence (e.g., a magnetic particle-labeled antibody targeting TPK1 protein, a biotin-labeled antibody targeting TPK1 protein, etc.).
   E7. The kit according to any one of clauses E1-E6, wherein the kit can be used for chemiluminescence detection.
   E8. The kit according to any one of clauses E1-E7, wherein the kit is compatible with fully automated chemiluminescence detection instruments or automated/digital detection platforms.
Clause set F:
   F1. A test strip or test card for distinguishing or differentially diagnosing Alzheimer's disease from other neurodegenerative diseases, wherein the test strip or test card comprises a portion for receiving a sample from a subject and a portion coated with a reagent capable of specifically binding to TPK1 protein; wherein the subject is suspected of having a neurodegenerative disease but it is difficult to determine whether the neurodegenerative disease is Alzheimer's disease, and wherein the biomarker is TPK1 protein, and a decrease in the level of TPK1 protein compared to a reference value suggests that the subject has Alzheimer's disease.
   F2. The test strip or test card according to clause F1, wherein the sample is a blood sample, preferably a human whole blood sample, more preferably a human whole blood cell sample.
   F3. The test strip or test card according to any one of clauses F1-F2, wherein the reference value is the level of TPK1 protein in a sample from a healthy subject (or a non-AD subject, or a non-AD elderly subject) or from a standard sample.
   F4. The test strip or test card according to any one of clauses F1-F3, wherein the level of TPK1 protein is determined by a method selected from: immunoassays, including competitive immunoassays, non-competitive immunoassays, enzyme-linked immunosorbent assays (ELISA), immunohistochemical assays, chemiluminescent assays (preferably magnetic particle chemiluminescence), Western blot assays, and Dot blot assays; mass spectrometry assays; optionally, the level of TPK1 protein is determined by chemiluminescent assays (preferably magnetic particle chemiluminescence).
   F5. The test strip or test card according to any one of clauses F1-F4, wherein the level of TPK1 protein is detected by a reagent capable of specifically binding to TPK1 protein; optionally, the level of TPK1 protein is detected by a binding partner targeting TPK1 protein; optionally, the level of TPK1 protein is detected by an antibody targeting TPK1 protein; optionally, the antibody is selected from monoclonal antibodies, polyclonal antibodies, antibody fragments, humanized antibodies, camelid antibodies, and chimeric antibodies.
   F6. The test strip or test card according to any one of clauses F1-F5, wherein the reagent may be further modified to carry a detectable label; preferably, the antibody may be further modified to carry a detectable label, such as a label detectable by chemiluminescence methods (e.g., magnetic particle-labeled antibodies targeting TPK1 protein, biotin-labeled antibodies targeting TPK1 protein, etc.).
   F7. The test strip or test card according to any one of clauses F1-F6, wherein when the test strip or test card is used to detect TPK1 protein, the test strip or test card comprises multiple portions coated with reagents capable of specifically binding to TPK1 protein (e.g., magnetic particle-labeled antibodies targeting TPK1 protein and biotin-labeled antibodies targeting TPK1 protein); optionally, the test strip or test card further comprises a detection portion for detecting whether the biomarker binds to the reagent (e.g., streptavidin-labeled alkaline phosphatase complexes and alkaline phosphatase substrates), and/or a portion for quality control.
   F8. The test strip or test card according to any one of clauses F1-F7, wherein the test strip or test card is suitable for chemiluminescence detection.
   F9. The test strip or test card according to any one of clauses F1-F8, wherein the test strip or test card is compatible with fully automated chemiluminescence detection instruments or automated/digital detection platforms.
Clause set G:
   G1. Use of a reagent for detecting a biomarker in a sample from a subject in the preparation of a test strip, test card, and/or kit for distinguishing or differentially diagnosing Alzheimer's disease and other neurodegenerative diseases, wherein the subject is suspected of having a neurodegenerative disease but it is difficult to determine whether the neurodegenerative disease is Alzheimer's disease, the biomarker is TPK1 protein, and a decrease in the level of TPK1 protein compared to a reference value suggests that the subject has Alzheimer's disease.
   G2. The use according to clause G1, wherein the sample is a blood sample, preferably a human whole blood sample, more preferably a human whole blood cell sample.
   G3. The use according to any one of clauses G1-G2, wherein the reference value is the level of TPK1 protein in a sample from a healthy subject (or a non-AD subject, or a non-AD elderly subject) or from a standard sample.
   G4. The use according to any one of clauses G1-G3, wherein the level of TPK1 protein is determined by a method selected from: immunoassays, including competitive immunoassays, non-competitive immunoassays, enzyme-linked immunosorbent assays (ELISA), immunohistochemical assays, chemiluminescence assays (preferably magnetic particle chemiluminescence), Western blot assays, and Dot blot assays; mass spectrometry assays; optionally, the level of TPK1 protein is determined by a chemiluminescence assay (preferably magnetic particle chemiluminescence).
   G5. The use according to any one of clauses G1-G4, wherein the level of TPK1 protein is detected by a reagent capable of specifically binding to TPK1 protein; optionally, the level of TPK1 protein is detected by a binding partner targeting TPK1 protein; optionally, the level of TPK1 protein is detected by an antibody targeting TPK1 protein; optionally, the antibody is selected from monoclonal antibodies, polyclonal antibodies, antibody fragments, humanized antibodies, camelid antibodies, and chimeric antibodies.
   G6. The use according to clause G5, wherein the reagent may be further modified to carry a detectable label; preferably, the antibody may be further modified to carry a detectable label, such as a label detectable by chemiluminescence methods (e.g., magnetic particle-labeled antibodies targeting TPK1 protein, biotin-labeled antibodies targeting TPK1 protein, etc.).
   G7. The use according to any one of clauses G1-G6, wherein when the test strip, test card, and/or kit is used to detect TPK1 protein, the test strip, test card, and/or kit comprises multiple portions coated with reagents capable of specifically binding to TPK1 protein (e.g., magnetic particle-labeled antibodies targeting TPK1 protein and biotin-labeled antibodies targeting TPK1 protein); optionally, the test strip or test card further comprises a detection portion for detecting whether the biomarker binds to the reagent (e.g., streptavidin-labeled alkaline phosphatase complexes and alkaline phosphatase substrates), and/or a portion for quality control.
   G8. The use according to any one of clauses G1-G7, wherein the test strip, test card, and/or kit is suitable for chemiluminescence detection.
   G9. The use according to any one of clauses G1-G8, wherein the test strip, test card, and/or kit is compatible with fully automated chemiluminescence detection instruments or automated/digital detection platforms.
Clause set H:
   H1. Use of a biomarker as a testing standard in a method or kit for distinguishing or differentially diagnosing Alzheimer's disease and other neurodegenerative diseases, wherein the biomarker is TPK1 protein.
   H2. The biomarker according to clause H1, wherein the biomarker is contained in a blood sample, preferably a human whole blood sample, more preferably a human whole blood cell sample.
   H3. The biomarker according to any one of clauses H1-H2, wherein the reference value is the level of TPK1 protein in a sample from a healthy subject (or a non-AD subject, or a non-AD elderly subject) or from a standard sample.
   H4. The biomarker according to any one of clauses H1-H3, wherein the level of TPK1 protein is determined by a method selected from: immunoassays, including competitive immunoassays, non-competitive immunoassays, enzyme-linked immunosorbent assays (ELISA), immunohistochemical assays, chemiluminescence assays (preferably magnetic particle chemiluminescence), Western blot assays, and Dot blot assays; mass spectrometry assays; optionally, the level of TPK1 protein is determined by a chemiluminescence assay (preferably magnetic particle chemiluminescence).
   H5. The biomarker according to any one of clauses H1-H4, wherein the level of TPK1 protein is detected by a reagent capable of specifically binding to TPK1 protein; optionally, the level of TPK1 protein is detected by a binding partner targeting TPK1 protein; optionally, the level of TPK1 protein is detected by an antibody targeting TPK1 protein; optionally, the antibody is selected from monoclonal antibodies, polyclonal antibodies, antibody fragments, humanized antibodies, camelid antibodies, and chimeric antibodies.
   H6. The biomarker according to clause H5, wherein the reagent may be further modified to carry a detectable label; preferably, the antibody may be further modified to carry a detectable label, such as a label detectable by chemiluminescence methods (e.g., magnetic particle-labeled antibodies targeting TPK1 protein, biotin-labeled antibodies targeting TPK1 protein, etc.).
Clause set I:
   I1. A method for treating a subject with Alzheimer's disease, comprising the steps of:
   a. diagnosing a subject suspected of having Alzheimer's disease using the method, composition, test strip, test card, and/or kit according to clause sets A-H, and
   b. administering a therapeutically effective amount of a drug for treating Alzheimer's disease to the subject, or performing an Alzheimer's disease-related therapy on the subject.
Clause set J:
   J1. A method for determining the likelihood of a subject having Alzheimer's disease, wherein the subject is suspected of having a neurodegenerative disease but it is difficult to determine whether the neurodegenerative disease is Alzheimer's disease, wherein the method comprises determining the level of TPK1 protein in a sample from the subject and comparing it to a reference value, wherein a decrease in the level of TPK1 protein compared to the reference value indicates a higher likelihood of the subject having Alzheimer's disease than other neurodegenerative diseases.
   J2. The method according to clause J1, wherein the sample is a blood sample, preferably a human whole blood sample, more preferably a human whole blood cell sample.
   J3. The method according to any one of clauses J1-J2, wherein the reference value is the level of TPK1 protein in a sample from a healthy subject (or a non-AD subject, or a non-AD elderly subject) or from a standard sample.
   J4. The method according to any one of clauses J1-J3, wherein the level of TPK1 protein is determined by a method selected from: immunoassays, including competitive immunoassays, non-competitive immunoassays, enzyme-linked immunosorbent assays (ELISA), immunohistochemical assays, chemiluminescence assays (preferably magnetic particle chemiluminescence), Western blot assays, and Dot blot assays; mass spectrometry assays; optionally, the level of TPK1 protein is determined by a chemiluminescence assay (preferably magnetic particle chemiluminescence).
   J5. The method according to any one of clauses J1-J4, wherein the level of TPK1 protein is detected by a reagent capable of specifically binding to TPK1 protein; optionally, the level of TPK1 protein is detected by a binding partner targeting TPK1 protein; optionally, the level of TPK1 protein is detected by an antibody targeting TPK1 protein; optionally, the antibody is selected from monoclonal antibodies, polyclonal antibodies, antibody fragments, humanized antibodies, camelid antibodies, and chimeric antibodies.
   J6. The method according to clause J5, wherein the reagent may be further modified to carry a detectable label; optionally, the antibody may be further modified to carry a detectable label, such as a label detectable by chemiluminescence methods (e.g., magnetic particle-labeled antibodies targeting TPK1 protein, biotin-labeled antibodies targeting TPK1 protein, etc.).
Clause set K:
   K1. A method for determining the likelihood of a subject having a neurodegenerative disease other than Alzheimer's disease, wherein the subject is suspected of having a neurodegenerative disease but it is difficult to exclude the possibility that the neurodegenerative disease is not Alzheimer's disease, wherein the method comprises determining the level of TPK1 protein in a sample from the subject and comparing it to a reference value, wherein the level of TPK1 protein remaining unchanged or increased compared to the reference value indicates a lower likelihood of the subject having Alzheimer's disease.
   K2. The method according to clause K1, wherein the sample is a blood sample, preferably a human whole blood sample, more preferably a human whole blood cell sample.
   K3. The method according to any one of clauses K1-K2, wherein the reference value is the level of TPK1 protein in a sample from a healthy subject (or a non-AD subject, or a non-AD elderly subject) or from a standard sample.
   K4. The method according to any one of clauses K1-K3, wherein the level of TPK1 protein is determined by a method selected from: immunoassays, including competitive immunoassays, non-competitive immunoassays, enzyme-linked immunosorbent assays (ELISA), immunohistochemical assays, chemiluminescence assays (preferably magnetic particle chemiluminescence), Western blot assays, and Dot blot assays; mass spectrometry assays; optionally, the level of TPK1 protein is determined by a chemiluminescence assay (preferably magnetic particle chemiluminescence).
   K5. The method according to any one of clauses K1-K4, wherein the level of TPK1 protein is detected by a reagent capable of specifically binding to TPK1 protein; optionally, the level of TPK1 protein is detected by a binding partner targeting TPK1 protein; optionally, the level of TPK1 protein is detected by an antibody targeting TPK1 protein; optionally, the antibody is selected from monoclonal antibodies, polyclonal antibodies, antibody fragments, humanized antibodies, camelid antibodies, and chimeric antibodies.
   K6. The method according to clause K5, wherein the reagent may be further modified to carry a detectable label; optionally, the antibody may be further modified to carry a detectable label, such as a label detectable by chemiluminescence methods (e.g., magnetic particle-labeled antibodies targeting TPK1 protein, biotin-labeled antibodies targeting TPK1 protein, etc.).
Clause set L:
   L1. An immunoassay composition, comprising TPK1 protein from a sample of a subject and an effective amount of a reagent (preferably a binding partner) for detecting the level of TPK1 protein, and/or comprising a complex formed by TPK1 protein from a sample of a subject and an effective amount of a reagent (preferably a binding partner) for detecting the level of TPK1 protein, wherein the subject is suspected of having a neurodegenerative disease but it is difficult to determine whether the neurodegenerative disease is Alzheimer's disease, and wherein a decrease in the level of TPK1 protein in the immunoassay composition compared to a reference value suggests that the subject has Alzheimer's disease.
   L2. A container comprising an immunoassay composition, wherein the immunoassay composition comprises TPK1 protein from a sample of a subject and an effective amount of a reagent (preferably a binding partner) for detecting the level of TPK1 protein, and/or comprising a complex formed by TPK1 protein from a sample of a subject and an effective amount of a reagent (preferably a binding partner) for detecting the level of TPK1 protein, wherein the subject is suspected of having a neurodegenerative disease but it is difficult to determine whether the neurodegenerative disease is Alzheimer's disease, and wherein a decrease in the level of TPK1 protein in the immunoassay composition in the container compared to a reference value suggests that the subject has Alzheimer's disease.
   L3. The composition according to clause L1 or the container according to clause L2, wherein the sample is a blood sample, preferably a human whole blood sample, more preferably a human whole blood cell sample.
   L4. The composition according to any one of clauses L1-L3 or the container according to any one of clauses L2-L3, wherein the reference value is the level of TPK1 protein in a sample from a healthy subject (or a non-AD subject, or a non-AD elderly subject) or from a standard sample.
   L5. The composition according to any one of clauses L1-L4 or the container according to any one of clauses L2-L4, wherein the level of TPK1 protein is determined by a method selected from: immunoassays, including competitive immunoassays, non-competitive immunoassays, enzyme-linked immunosorbent assays (ELISA), immunohistochemical assays, chemiluminescence assays (preferably magnetic particle chemiluminescence), Western blot assays, and Dot blot assays; mass spectrometry assays; optionally, the level of TPK1 protein is determined by a chemiluminescence assay (preferably magnetic particle chemiluminescence).
   L6. The composition according to any one of clauses L1-L5 or the container according to any one of clauses L2-L5, wherein the level of TPK1 protein is detected by a reagent capable of specifically binding to TPK1 protein; optionally, the level of TPK1 protein is detected by a binding partner targeting TPK1 protein; optionally, the level of TPK1 protein is detected by an antibody targeting TPK1 protein; optionally, the antibody is selected from monoclonal antibodies, polyclonal antibodies, antibody fragments, humanized antibodies, camelid antibodies, and chimeric antibodies.
   L7. The composition according to any one of clauses L1-L6 or the container according to any one of clauses L2-L6, wherein the reagent may be further modified to carry a detectable label; optionally, the antibody may be further modified to carry a detectable label, such as a label detectable by chemiluminescence methods (e.g., magnetic particle-labeled antibodies targeting TPK1 protein, biotin-labeled antibodies targeting TPK1 protein, etc.).
   L8. The composition according to any one of clauses L1-L7 or the container according to any one of clauses L2-L7, wherein the container may be in the form of a test tube, well, vial, or the like.

## Claims

1. Use of a reagent for detecting a biomarker in a sample from a subject in the preparation of a test strip, test card, and/or kit for distinguishing or differentially diagnosing Alzheimer's disease and other neurodegenerative diseases, wherein the subject is suspected of having a neurodegenerative disease but it is difficult to determine whether the neurodegenerative disease is Alzheimer's disease, the biomarker is TPK1 protein, and a decrease in the level of TPK1 protein compared to a reference value suggests that the subject has Alzheimer's disease.

2. The use according to claim 1, wherein the sample is a blood sample, preferably a human whole blood sample, more preferably a human whole blood cell sample.

3. The use according to any one of claims 1-2, wherein the reference value is the level of TPK1 protein in a sample from a healthy subject (or a non-AD subject, or a non-AD elderly subject) or from a standard sample.

4. The use according to any one of claims 1-3, wherein the level of TPK1 protein is determined by a method selected from: immunoassays, including competitive immunoassays, non-competitive immunoassays, enzyme-linked immunosorbent assays (ELISA), immunohistochemical assays, chemiluminescence assays (preferably magnetic particle chemiluminescence), Western blot assays, and Dot blot assays; mass spectrometry assays; optionally, the level of TPK1 protein is determined by a chemiluminescence assay (preferably magnetic particle chemiluminescence).

5. The use according to any one of claims 1-4, wherein the level of TPK1 protein is detected by a reagent capable of specifically binding to TPK1 protein; optionally, the level of TPK1 protein is detected by a binding partner targeting TPK1 protein; optionally, the level of TPK1 protein is detected by an antibody targeting TPK1 protein; optionally, the antibody is selected from monoclonal antibodies, polyclonal antibodies, antibody fragments, humanized antibodies, camelid antibodies, and chimeric antibodies.

6. The use according to claim 5, wherein the reagent may be further modified to carry a detectable label; preferably, the antibody may be further modified to carry a detectable label, such as a label detectable by chemiluminescence methods (e.g., magnetic particle-labeled antibodies targeting TPK1 protein, biotin-labeled antibodies targeting TPK1 protein, etc.).

7. The use according to any one of claims 1-6, wherein when the test strip, test card, and/or kit is used to detect TPK1 protein, the test strip, test card, and/or kit comprises multiple portions coated with reagents capable of specifically binding to TPK1 protein (e.g., magnetic particle-labeled antibodies targeting TPK1 protein and biotin-labeled antibodies targeting TPK1 protein); optionally, the test strip or test card further comprises a detection portion for detecting whether the biomarker binds to the reagent (e.g., streptavidin-labeled alkaline phosphatase complexes and alkaline phosphatase substrates), and/or a portion for quality control.

8. The use according to any one of claims 1-7, wherein the test strip, test card, and/or kit is suitable for chemiluminescence detection.

9. The use according to any one of claims 1-8, wherein the test strip, test card, and/or kit is compatible with fully automated chemiluminescence detection instruments or automated/digital detection platforms.
